(19) Europäisches Patentamt
European Patent Office
Office européen des brevets

(11) Veröffentlichungsnummer: **0 199 986**
**B1**

(12) # EUROPÄISCHE PATENTSCHRIFT

(45) Veröffentlichungstag der Patentschrift: **25.04.90**

(21) Anmeldenummer: **86104008.7**

(22) Anmeldetag: **24.03.86**

(51) Int. Cl.[5]: **C 07 D 293/12,**
C 07 D 517/04, A 61 K 31/41
// (C07D517/04, 317:00, 293:00)

(54) **Neue Benzisoselenazolonyl-Derivate, Verfahren zu ihrer Herstellung und diese enthaltende pharmazeutische Präparate.**

(30) Priorität: **27.04.85 DE 3515273**
**27.04.85 DE 3515274**

(43) Veröffentlichungstag der Anmeldung:
**05.11.86 Patentblatt 86/45**

(45) Bekanntmachung des Hinweises auf die Patenterteilung:
**25.04.90 Patentblatt 90/17**

(84) Benannte Vertragsstaaten:
**AT BE CH DE FR GB IT LI LU NL SE**

(56) Entgegenhaltungen:
**EP-A-0 154 851** **DE-A-3 027 075**
**DE-A-3 027 074** **DE-A-3 226 286**

**T.W. GREENE "Protective Groups in Organic Synthesis", 1981, Seite 175, John Wiley & Sons, New York, US;**

**Die Akte enthält technische Angaben, die nach dem Eingang der Anmeldung eingereicht wurden und die nicht in dieser Patentschrift enthalten sind.**

(73) Patentinhaber: **A. Nattermann & Cie. GmbH**
**Nattermannallee 1**
**D-5000 Köln 30 (DE)**

(72) Erfinder: **Welter, André, Dr.**
**Grand' Route 84**
**B-4610 Beyne-Hevsay (BE)**
Erfinder: **Lambert, Christian**
**8 Place Reine Astrid**
**B-4540 Visé (BE)**
Erfinder: **Dereu, Norbert, Dr.**
**An der Holzhecke 11**
**D-5020 Frechen-Bachem (DE)**
Erfinder: **Hüther, Andrea, Dr.**
**Brauweiler 133**
**D-5000 Köln 41 (DE)**
Erfinder: **Etschenberg, Eugen, Dr.**
**Hirseweg 10**
**D-5000 Köln 41 (DE)**

(74) Vertreter: **Patentanwaltsbüro Cohausz & Florack**
**Schumannstrasse 97**
**D-4000 Düsseldorf 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 199 986 B1

**Beschreibung**

Die vorleigende Erfindung betrifft neue Benzisoselenazolonyl-Derivate, Verfahren zu ihrer Herstellung und diese Verbindung enthaltende pharmazeutische Zubereitungen.

Benzisoselenazolone mit antiarteriosklerotischen und entzündungshemmenden Eigenschaften sind bereits mehrfach beschrieben worden, so z.B. in DE—A—30 27 073 = US—A—4,352,799; DE—A—30 27 074; DE—A—30 27 075 = US—A—4,418,069; DE—A—32 26 284; DE—A—32 26 286.

Es wurde nun gefunden, daß Benzisoselenazolonyl-Derivate der allgemeinen Formel I

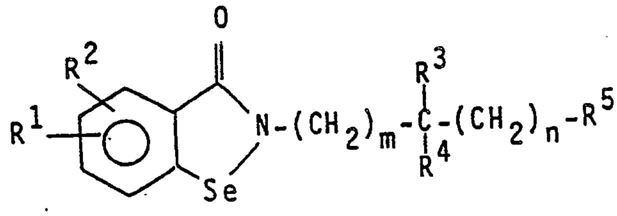

I

worin

$R^1$, $R^2$ gleich oder verschieden sind und unabhängig voneinander Wasserstoff, Halogen, $C_{1-4}$-Alkyl, $C_{1-4}$-Alkoxy, Trifluormethyl, Nitro oder zusammen Methylendioxy bedeuten und

$R^3$ für Wasserstoff, $C_{1-4}$-Alkyl gerade oder verzweigt, Mercapto-$C_{1-2}$-alkyl, Methylthio-$C_{1-2}$-alkyl oder Phenylmethyl steht und

$R^4$ Wasserstoff oder Methyl bedeutet, während

$R^5$ für Wasserstoff, Methyl, $C_{3-8}$-Cycloalkyl, —COOH, —$CONH_2$, —CN oder —$COOR^6$ steht, wobei $R^6$ einen geraden oder verzweigten $C_{1-4}$-Alkylrest bedeutet und

m, n gleich oder verschieden sind und Null oder eine ganze Zahl von 1—8 sind, wobei die Alkylenkette —$(CH_2)_n$— bei n = 6 auch als 1,4-Cyclohexylengruppe vorliegen kann,

wertvolle pharmakologische Eigenschaften aufweisen. Ausgenommen hiervon sind die in der DE—OS 30 27 074 beschriebenen Verbindungen der Formel I, die als Rest

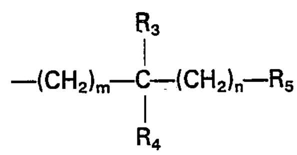

eine n-Alkylgruppe mit 1—4 C-Atomen haben und bei denen gleichzeitig $R^1$ und $R^2$ Wasserstoff ist.

Bevorzugt sind dabei Benzisoselenazolonyl-Derivate der Formel I, worin $R^1$, $R^2$ gleich oder verschieden sind und unabhängig voneinander Wasserstoff, Fluor, Chlor, Brom, Methyl, Methoxy, Trifluormethyl, Nitro oder zusammen Methylendioxy bedeuten und $R^3$ fur Wasserstoff, $C_{1-4}$-Alkyl gerade oder verzweigt, Mercapto-$C_{1-2}$-alkyl, Methylthio-$C_{1-2}$-alkyl oder Phenylmethyl steht und $R^4$ Wasserstoff oder Methyl bedeutet und $R^5$ für —COOH steht, während m und n Null sind.

Besonders bevorzugt sind dabei Verbindungen, bei denen $R^3$, $R^4$ beide Methyl und $R^5$ Wasserstoff oder Methyl bedeuten, während m und n Null sind.

Verbindungen der Formel I, die aufgrund unterschiedlicher Substituenten für $R^3$ und $R^4$ ein Chiralitätszentrum an dem entsprechenden Kohlenstoffatom besitzen, können je nach Art der verwendeten Ausgangssubstanzen als Racemate oder in form der D- oder L-Enantiomeren vorliegen. Falls eine Trennung der Racemate erwünscht ist, wird diese zweckmäßigerweise nach an sich bekannten Verfahren mit geeigneten optisch aktiven Basen über die Bildung von diastereomeren Salzen oder durch Chromatographie an optisch aktivem Säulenmaterial durchgeführt.

Erfindungsgemäße Verbindungen sind beispielsweise:

1,2-Benzisoselenazol-3(2H)-on-2-ylessigsäure
1,2-Benzisoselenazol-3(2H)-on-2-ylessigsäuremethylester
L-2-(1,2-Benzisoselenazol-3(2H)-on-2-yl)-propionsäure
L-2-(1,2-Benzisoselenazol-3(2H)-on-2-yl)-propionsäureamid
L-2-(6-Chlor-1,2-benzisoselenazol-3(2H)-on-2-yl)-propionsäure
L-2-(6-Fluor-1,2-benzisoselenazol-3(2H)-on-2-yl)-propionsäure
L-2-(6-Methyl-1,2-benzisoselenazol-3(2H)-on-2-yl)-propionsäure
L-2-(6-Trifluormethyl-1,2-benzisoselenazol-3(2H)-on-2-yl)-propionsäure
L-2-(7-Nitro-1,2-benzisoselenazol-3(2H)-on-2-yl)-propionsäure
L-2-(7-Methoxy-1,2-benzisoselenazol-3(2H)-on-2-yl)-propionsäure
L-2-(6,7-Methylendioxy-1,2-benzisoselenazol-3(2H)-on-2-yl)-propionsäure
2-(1,2-Benzisoselenazol-3(2H)-on-2-yl)-2-methylpropionsäure
2-(7-Methoxy-1,2-benzisoselenazol-3(2H)-on-2-yl)-2-methylpropionsäure

3-(1,2-Benzisoselenazol-3(2H)-on-2-yl)-propionsäure
4-(1,2-Benzisoselenazol-3(2H)-on-2-yl)-buttersäure
L-2-(1,2-Benzisoselenazol-3(2H)-on-2-yl)-3-methylthiopropionsäure
L-2-(1,2-Benzisoselenazol-3(2H)-on-2-yl)-4-methylthiobuttersäure
DL-2-(1,2-Benzisoselenazol-3(2H)-on-2-yl)-3-phenylpropionsäure
L-2-(1,2-Benzisoselenazol-3(2H)-on-2-yl)-3-methylbuttersäure
DL-2-(1,2-Benzisoselenazol-3(2H)-on-2-yl)-3-methylbuttersäure
L-2-(1,2-Benzisoselenazol-3(2H)-on-2-yl)-4-methylvaleriansäure
5-(1,2-Benzisoselenazol-3(2H)-on-2-yl)-valeriansäure
6-(1,2-Benzisoselenazol-3(2H)-on-2-yl)-capronsäure
8-(1,2-Benzisoselenazol-3(2H)-on-2-yl)-octansäure
10-(1,2-Benzisoselenazol-3(2H)-on-2-yl)-decansäure
12-(1,2-Benzisoselenazol-3(2H)-on-2-yl)-dodecansäure
14-(1,2-Benzisoselenazol-3(2H)-on-2-yl)-tetradecansäure
16-(1,2-Benzisoselenazol-3(2H)-on-2-yl)-hexadecansäure
18-(1,2-Benzisoselenazol-3(2H)-on-2-yl)-octadecansäure
trans-4-(1,2-Benzisoselenazol-3(2H)-on-2-ylmethyl)-cyclohexancarbonsäure
1,2-Benzisoselenazol-3(2H)-on-2-yl-acetonitril
2-Cyclopropylmethyl-1,2-benzisoselenazol-3(2H)-on
2-Cyclohexylmethyl-1,2-benzisoselenazol-3(2H)-on
2-tert-Butyl-7-methyl-1,2-benzisoselenazol-3(2H)-on
2-Cyclohexylmethyl-6-fluor-1,2-benzisoselenazol-3(2H)-on
2-Cyclohexylmethyl-7-methoxy-1,2-benzisoselenazol-3(2H)-on
2-Cycloheptylmethyl-1,2-benzisoselenazol-3(2H)-on
2-tert-Butyl-6-chlor-1,2-benzisoselenazol-3(2H)-on
5-Chlor-2-octyl-1,2-benzisoselenazol-3(2H)-on
2-tert-Butyl-5-nitro-1,2-benzisoselenazol-3(2H)-on
2-Cycloheptylmethyl-6,7-methylendioxy-1,2-benzisoselenazol-3(2H)-on
2-Pentyl-1,2-benzisoselenazol-3(2H)-on
2-Hexyl-1,2-benzisoselenazol-3(2H)-on
2-Octyl-1,2-benzisoselenazol-3(2H)-on
2-Decyl-1,2-benzisoselenazol-3(2H)-on
2-Dodecyl-1,2-benzisoselenazol-3(2H)-on
2-Hexadecyl-1,2-benzisoselenazol-3(2H)-on
2-Octadecyl-1,2-benzisoselenazol-3(2H)-on
2-tert-Butyl-1,2-benzisoselenazol-3(2H)-on
2-Isopropyl-1,2-benzisoselenazol-3(2H)-on
2-tert-Butyl-7-nitro-1,2-benzisoselenazol-3(2H)-on
2-Isopropyl-7-trifluormethyl-1,2-benzisoselenazol-3(2H)-on

Die erfindungsgemäßen Verbindungen der Formel I weisen wertvolle pharmakologische Eigenschaften auf. Zum Nachweis der entzündungshemmenden Wirkung wurde das Cobra-Venom-Factor(CVF)-Ödem ausgewählt, weil bekannt ist, daß sowohl Cyclooxygenase wie Lipoxgenase gemeinsam hemmende Substanzen, z.B. Phenidon, als auch immunregulierende Verbindungen, z.B. Levamisol, einen ausgeprägteren hemmenden Effekt im CVF-Ödem-Test zeigen als im Carragenin-Test (S. Leyck, E. Etschenberg, U. Hadding, J. Winkelmann, Agents and Actions *13*, 437—438, 1983). Das CVF-Ödem ist von der Aktivierung des Complementsystems abhängig, das eine wichtige Rolle in akuten und chronischen Entzündungsprozessen spielt, in denen es die Aktivität von Immunkomplexen beeinflußt. Besonders hervonzuheben ist die überlegene Wirksamkeit im Vergleich zu der Referenzsubstanz Ebselen, INN (1-Phenyl-1,2-benzisoselenazol-3(2H)-on).

$ED_{50}$ (mg/kg p.o.)-Werte im CVF-Ödem

| Substanz | $ED_{50}$ (mg/kg p.o.) |
|---|---|
| Ebselen (Referenzsubstanz) | 56,2 |
| D,L-2-(1,2-Benzisoselenazol-3(2H)-on-2-yl)-3-methylbuttersäure | 5,0 |
| 2-Isopropyl-1,2-benzisoselenazol-3(2H)-on | 10,3 |
| 2-tert-Butyl-1,2-benzisoselenazol-3(2H)-on | 17,7 |

Die erfindungsgemäßen Verbindungen der Formel I können zur Behandlung zahlreicher Krankheiten verwendet werden, wie z.B. zur Prophylaxe und Therapie von Infektionskrankheiten, zur Stimulierung des Immunsystems oder bei Selenmangelkrankheiten, wie sie bei W. Kraus und P. Oehm, Das Deut, Gesundheitswesen, 1979, 34 (37), 1713—1718 und 1979, 35 (37), 1769—1773, definiert werden.

Die Benzisoselenazolonyl-Derivate der Formel I zeichnen sich jedoch besonders durch antiarteriosklerotische und entzündungshemmende Eigenschaften aus. Sie eignen sich daher besonders zur Therapie von rheumatischen Krankheiten, wie z.B. Arthrosen oder chronischer Polyarthritis, zur Lebertherapie, zur Behandlung von Hautkrankheiten wie Psoriasis. Die neuen Verbindungen zeichnen sich durch eine sehr gute Verträglichkeit aus, da sie untoxisch sind und im Gegensatz zu bekannten enzündungshemmenden Therapeutika keinerlei Ulcusbildung oder gastrointestinale Irritationen zeigen.

Die Herstellung der erfindungsgemäßen Verbindungen erfolgt nach an sich bekannten Verfahren. Dabei werden o-Chlorselenobenzoesäurechloride der allgemeinen Formel II

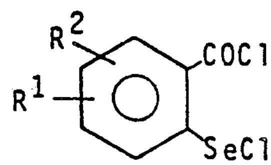

II

worin $R^1$, $R^2$ die in Formel I angegebenen Bedeutungen haben, mit der Aminogruppe von Verbindungen der Formel III

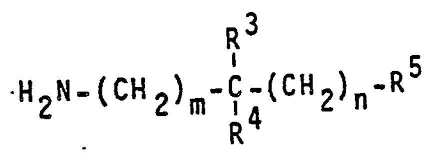

III

worin $R^3$, $R^4$, $R^5$ die in Formel I angegebenen Bedeutungen haben, unter Ringschlußbedingungen zu den Benzisoselenazolonyl-Derivaten der Formel I umgesetzt, wobei gegebenenfalls im Molekül der Verbindung III anwesende reaktionsfähige Gruppen, z.B. $R^5$ = COOH vorher in üblicher geeigneter Weise geschützt werden.

Besonders hervorzuheben ist ein Verfahren zur Herstellung von Verbindungen gemäß den Ansprüchen 1—2, in denen $R^5$ eine Carboxylgruppe bedeutet, dadurch gekennzeichnet, daß in einem Eintopfverfahren die Aminocarbonsäuren der Formel III in einem geeigneten Lösungsmittel unter Rückflußbedingungen in die Trimethylsilylester umgewandelt werden, diese nach Abkühlung auf 0°C unter Zugabe des entsprechenden o-Chlorselenobenzoesäurechlorids und der notwendigen Menge Triethylamin zu den Benzisoselenazolonylalkansäuretrimethylsilylestern kondensiert werden und aus den erhaltenen Produkten die Trimethylsilylesterschutzgruppe in üblicher Weise unter Erhalt der freien Carbonsäuren der Formel I mit $R^5$ = COOH abspaltet.

Die Herstellung der entsprechenden o-Chlorselenobenzoesäurechloride erfolgt nach dem Verfahren von A. Ruwet und M. Renson, Bull. Soc. Chim. Belg. 1966, *76*, 157—163 und von W. R. Gaythwaite, J. Kenyon und H. Phillips, J. Chem. Soc. (1928), 2280.

Als Ausgangsverbindungen der Formel II kommen z.B. die folgenden Verbindungen in Betracht:

2-Chlorseleno-4-chlorbenzoylchlorid
2-Chlorseleno-4-fluorbenzoylchlorid
2-Chlorseleno-4-brombenzoylchlorid
2-Chlorseleno-4-methylbenzoylchlorid
2-Chlorseleno-4-methoxybenzoylchlorid
2-Chlorseleno-4-trifluormethylbenzoylchlorid
2-Chlorseleno-5-chlorbenzoylchlorid
2-Chlorseleno-5-nitrobenzoylchlorid
2-Chlorseleno-3-methoxybenzoylchlorid
2-Chlorseleno-3-nitrobenzoylchlorid
2-Chlorseleno-3,4-methylenedioxybenzoylchlorid

Die Ausgangsverbindungen der Formel III sind bekannte Verbindungen, wie z.B.:

Aminoessigsäure, Aminoessigsäuremethylester, L-2-Aminopropionsäure, D-2-Aminopropionsäure, D,L-2-Aminopropionsäure, DL-2-Aminopropionsäureethylester, DL-2-Aminopropionsäure-tert-butylester, L-2-Aminopropionsäureamid, 2-Amino-iso-buttersäure, 3-Aminopropionsäure, 3-Aminopropionsäuremethylester, 4-Aminobuttersäure, 4-Aminobuttersäureethylester, DL-2-Aminobuttersäureethylester, L-2-Amino-3-mercapto-propionsäure, L-2-Amino-2-methylthio-propionsäure, L-2-Amino-4-methylthio-buttersäure, DL-2-Amino-3-phenylpropionsäure, L-2-Amino-3-methyl-buttersäure, L-2-Amino-4-methylvaleriansäure, 5-Aminovaleriansäure, 6-Aminocapronsäure, 8-Aminooctansäure, 10-Aminodecansäure, 12-Aminododecansäure, 14-Aminotetradecansäure, 16-Aminohexadecansäure, 18-Aminooctadecansäure, trans-4-Aminomethylcyclohexancarbonsäure, Aminoacetonitril, Aminomethylcyclopropan, Aminomethyl-cyclohexan, Aminomethyl-cyclooctan, Pentylamin, Hexylamin, Octylamin, Decylamin, Dodecylamin, Hexadecylamin, Octadecylamin, Isopropylamin, tert-Butylamin.

Die vorliegende Erfindung betrifft ebenfalls pharmazeutisch Präparate, welche Verbindungen der Formel I enthalten. Bei den erfindungsgemäßen pharmazeutischen Präparaten handelt es sich um solche zur enteralen, wie oralen oder rektalen sowie parenteralen Verabreichung, welche die pharmazeutischen Wirkstoffe allein oder zusammen mit einem üblichen pharmazeutisch anwendbaren Trägermaterial enthalten. Vorteilhafterweise liegt die pharmazeutische Zubereitung des Wirkstoffes in Form von Einzeldosen vor, die auf die gewünschte Verabreichung abgestimmt sind, wie z.B. Tabletten, Dragees, Kapseln, Suppositorien, Granulate, Lösungen, Emulsionen oder Suspensionen. Die Dosierung der Substanzen liegt üblicherweise zwischen 10 und 1000 mg pro Tag, vorzugsweise zwischen 30 und 300 mg pro Tag und kann in einer Dosis oder mehreren Teildosen, vorzugsweise in zwei bis drei Teildosen pro Tag, verabreicht werden.

Die Herstellung der erfindungsgemäßen Verbindungen wird durch die folgenden Beispiele erläutert.

Die angegebenen Schmelzpunkte wurden in einem Büchi 510-Schmelzpunktbestimmungsapparat gemessen und sind mit °C angegeben und nicht korrigiert.

Beispiel 1

3-(1,2-Benzisoselenazol-3(2H)-on-2-yl)-propionsäure.

Zu einer Lösung von 8,9 g (0,1 Mol) 3-Aminopropionsäure in 200 ml Chloroform und 40 ml Acetonitril (absolut) werden 10,86 g (0,1 Mol) chlortrimethylsilan zugegeben. Die Mischung wird 3 Stunden unter Rückfluß erhitzt, dann auf 0°C abgekühlt. Nach Zugabe von 22,7 g (0,09 Mol) o-Chlorselenobenzoesäurechlorid werden unter Rühren 30,3 g (0,3 Mol) Triethylamin zugetropft. Die Mischung wird 2 Stunden weitergerührt und anschließend mit 200 ml 5%iger wäßriger Citronensäure-Lösung und 200 ml 10%iger Natriumhydrogencarbonat-Lösung gewaschen. Nach Ansäuren der wäßrigen Phase mit verdünnter Salzsäure wird der Niederschlag abgesaugt und aus Methanol umkristallisiert.

Ausbeute: 10,3 g (38% d.Th.) Fp. 188—190°C

Beispiel 2

4-(1,2-Benzisoselenazol-3(2H)-on-2-yl)-buttersäure.

Analog Beispiel 1 aus:

22,7 o-Chlorselenobenzoesäurechlorid
10,31 g 4-Aminobuttersäure

Ausbeute: 9,97 g (35% d.Th.) Fp. 142—145°C

Beispiel 3

6-(1,2-Benzisoselenazol-3(2H)-on-2-yl)-capronsäure.

Analog Beispiel 1 aus:

22,7 g o-Chlorselenobenzoesäurechlorid
13,1 g 6-Aminocapronsäure

Ausbeute: 13,24 g (42,3% d.Th.) Fp. 100—103°C

Beispiel 4

1,2-Benzisoselazol-3(2H)-on-2-ylessigsäure.

Analog Beispiel 1 aus:

11,35 g o-Chlorselenobenzoesäurechlorid
3,75 g Aminoessigsäure

Ausbeute: 4,76 g (39,5% d.Th.) Fp. 197—200°C

Beispiel 5

trans-4-(1,2-Benzisoselenazol-3(2H)-on-2-yl-methyl)-cyclohexancarbonsäure.

In eine Lösung von 10 g (0,0636 Mol) trans-4-(Aminomethyl)-cyclohexancarbonsäure und 8 g (0,2 Mol) Natriumhydroxid in 100 ml Wasser werden unter Eiskühlung 16,2 g (0,0636 Mol) o-Chlorselenobenzosäurechlorid zugegeben. Die Mischung wird über Nacht bei Raumtemperatur weitergerührt. Nach dem Ansäuern mit verdünnter Salzsäure auf pH 2 wird der Niederschlag abgesaugt und über eine Kieselgelsäule (Elutionsmittel Dichlormethan/Methanol 9:1) gereinigt.

Ausbeute: 6,2 g (28,8%) Fp. 199—201°C

Beispiel 6

8-(1,2-Benzisoselenazol-3(2H)-on-2-yl)-octansäure.

Analog Beispiel 1 aus:

11,35 g o-Chlorselenobenzoesäurechlorid
7,96 g 8-Aminooctansäure

Ausbeute: 6 g (40%) Fp. 132—135°C

Beispiel 7

12-(1,2-Benzisoselenazol-3(2H)-on-2-yl)-dodecansäure.

Analog Beispiel 1 aus:

11,35 g o-Chlorselenobenzoesäurechlorid
10,76 g Aminododecansäure

Ausbeute: 8,18 g (46,3% d.Th.) Fp. 104—105°C

Beispiel 8

DL-2-(1,2-Benzisoselenazol-3(2H)-on-2-yl)-phenyl-3-propionsäure.

Analog Beispiel 1 aus:

11,35 g o-Chlorselenobenzoesäurechlorid
8,26 g DL-Phenylalanin

Ausbeute: 4,47 g (31,9%) Fp. 165°C

Beispiel 9

(1,2-Benzisoselenazol-3(2H)-on-2-yl)-essigsäurenitril.

Analog Beispiel 1 aus:

11,35 g o-Chlorselenobenzoesäurechlorid
2,79 g 8-Aminoacetonitril

Ausbeute: 3,6 g (34%) Fp. 164—167°C

Beispiel 10

L-2-(1,2-Benzisoselenazol-3(2H)-on-2-yl)-propionsäure.

Analog Beispiel 1 aus:

11,35 g o-Chlorselenobenzoesäurechlorid
4,45 g Aminopropionsäure

Ausbeute: 1,68 g (14%) Fp. 211—215°C

Beispiel 11

4-(7-Methyl-(1,2-Benzisoselenazol-3(2H)-on-2-yl)-buttersäure.

Analog Beispiel 1 aus:

2,69 g 2-Chlorseleno-3-methylbenzoesäurechlorid
1,03 g 4-Aminobuttersäure

Ausbeute: 0,98 g (44,7%) Fp. 168—170°C

Beispiel 12

trans-4-(1,2-Benzisoselenazol-3(2H)-on-2-ylmethyl)-cyclohexancarbonsäuremethylester.

Zu einer Lösung von 4,09 (0,02 Mol) trans-4-Aminomethylcyclohexancarbonsäuremethylester Hydrochlorid und 4,96 g (0,06 M) Natriumhydrogencarbonat in 150 ml Wasser werden bei 5°C 5 g (0,02 Mol) o-Chlorselenobenzoesäurechlorid gelöst in 100 ml Diisopropylether innerhalb 40 Min. Zugetropft. Der enstandene Niederschlag wird abgesaugt, mit wenig Diethylether und mit Wasser gewaschen. Der unlösliche Rückstand wird aus Ethanol-Ether umkristallisiert.

Ausbeute: 2,6 (37,2%) Schm. 138°C

Beispiel 13

1,2-Benzisoselenazol-3(2H)-on-2-ylessigsäureethylester.

Analog Beispiel 12 aus:

3,06 g o-Chlorselenobenzoesäurechlorid
1,68 g Aminoessigsäureethylester Hydrochlorid

Ausbeute: 1,48 g (43,2%) Fp. 128—124°C

Beispiel 14

2-(1,2-Benzisoselenazol-3(2H)-on-2-yl)-2-methylpropionsäure.

Analog Beispiel 1 aus:

4,54 g 2-Chlorselenobenzoesäurechlorid
2,06 g 2-Amino-2-methylpropionsäure

Ausbeute: 4 g (70% d.Th.) Fp. 215°C

Beispiel 15

DL-2-(1,2-Benzisoselenazol-3(2H)-on-2-yl)-3-methylbuttersäure.

Analog Beispiel 1 aus:

5,1 g 2-Chlorselenobenzoesäurechlorid
2,5 g D,L-Valin

Ausbeute: 4,8 g (80% d.Th.) Fp. 177—179°C

Beispiel 16

L-2-(1,2-Benzisoselenazol-3(2H)-on-2-yl)-3-methylbuttersäure.

Analog Beispiel 1 aus:

5,1 g 2-Chlorselenobenzoesäurechlorid

2,5 g L-Valin

Ausbeute: 4,6 g (77,2% d.Th.) Fp. 165°C

Beispiel 17

D-2-(1,2-Benzisoselenazol-3(2H)-on-2-yl)-3-methylbuttersäure.

Analog Beispiel 1 aus:

5,1 g 2-Chlorselenobenzoesäurechlorid

2,5 g D-Valin

Ausbeute: 4,8 g (80,5% d.Th.) Fp. 164—166°C

Beispiel 18

L-2-(1,2-Benzisoselenazol-3(2H)-on-2-yl)-3-methylvaleriansäure.

Analog Beispiel 1 aus:

4,54 g 2-Chlorselenobenzoesäurechlorid

2,62 g 2-Amino-3-methyl-valeriansäure

Ausbeute: 2,6 g (42% d.Th.) Fp. 158—160°C

Beispiel 19

L-2-(1,2-Benzisoselenazol-3(2H)-on-2-yl)-4-methylbuttersäure.

Analog Beispiel 1 aus:

10 g 2-Chlorselenobenzoesäurechlorid

6,5 g L-Methionin

Ausbeute: 1 g (13% d.Th.) Fp. 171—172°C

Beispiel 20

2-Cyclohexylmethyl-1,2-benzisoselenazol-3(2H)-on.

2,2 g (0,02 Mol) Aminomethylcyclohexan und 3,93 g (0,04 Mol) Triäthylamin werden in 30 ml Dichlormethan gelöst und unter Rühren und Eiskühlung in einer Stickstoffatmosphäre zu einer Lösung von 4,93 g (0,02 Mol) o-Chlorselenobenzoesäurechlorid in 70 ml Dichlormethan zugetropft. Die Lösung wird über Nacht bei Raumtemperatur weitergerührt und anschließend eingeengt. Der Rückstand wird mit Wasser versetzt und mit verdünnter Salzsäure auf pH 2 gebracht. Der unlösliche Feststoff wird abgesaugt und zweimal aus Ethanol-Wasser umkristallisiert.

Ausbeute: 2,55 g (44,7% d.Th.) Fp. 155—156°C

Beispiel 21

2-tert-Butyl-1,2-benzisoselenazol-3(2H)-on.

Zu einer Lösung von 1,46 g (0,02 Mol) tert-Butylamin in 36,2 ml 1,1 N NaOH werden bei 5°C 5,08 g (0,02 Mol) o-Chlorselenobenzoesäurechlorid gelöst in 38 ml Diisopropylether innerhalb einer Stunde zugetropft. Der entstandene Niederschlag wird abgesaugt, mit wenig Diethylether und mit Wasser gewaschen. Der unlösliche Rückstand wird aus Tetrahydrofuran umkristallisiert.

Ausbeute: 2,2 g (44% d.Th.) Fp. 153—154°C

Beispiel 22

2-Hexyl-1,2-benzisoselenazol-3(2H)-on.

Analog Beispiel 20 aus:

10 g 2-Chlorselenobenzoesäurechlorid

13 g Hexylamin

Ausbeute: 5,78 g (51,6% d.Th.) Fp. 90—94°C

Beispiel 23

2-Isopropyl-1,2-benzisoselenazol-3(2H)-on.

Analog Beispiel 21 aus:

12,7 g 2-Chlorselenobenzoesäurechlorid

2,95 g Isopropylamin

Ausbeute: 5,5 g (45,8% d.Th.) Fp. 105—106°C

Beispiel 24

2-Octyl-1,2-benzisoselenazol-3(2H)-on.

Analog Beispiel 21 aus:

2,54 g 2-Chlorselenobenzoesäurechlorid

1,29 g Octylamin

Ausbeute: 1,46 g (46,9% d.Th.) Fp. 65°C

Beispiel 25

2-Dodecyl-1,2-benzisoselenazol-3(2H)-on.

Analog Beispiel 21 aus:

2,54 g 2-Chlorselenobenzoesäurechlorid

1,85 g Dodecylamin

Ausbeute: 1,63 g (44,4% d.Th.) Fp. 77—79°C

Beispiel 26

6-Chlor-2-tert-butyl-1,2-benzisoselenazol-3(2H)-on.

Analog Beispiel 21 aus:

5,79 g 2-Chlorselenobenzoesäurechlorid

1,46 g tert-Butylamin

Ausbeute: 2,1 g (36,2% d.Th.) Fp. 245—247°C

Beispiel 27

5-Chlor-2-octyl-1,2-benzisoselenazol-3(2H)-on.

Analog Beispiel 21 aus:

5,79 g 5-Chlor-2-Chlorselenobenzoesäurechlorid

2,585 g Octylamin

Ausbeute: 2,85 g (41,3% d.Th.) Fp. 224—226°C

Beispiel 28

2-tert-Butyl-5-nitro-1,2-benzisoselenazol-3(2H)-on.

Analog Beispiel 21 aus:

6,0 g 2-Chlorseleno-5-nitro-benzoesäurechlorid

1,46 g tert-Butylamin

Ausbeute: 2,46 g (44% d.Th.) Fp. 210—212°C

Beispiel 29

2-tert-Butyl-7-methyl-1,2-benzisoselenazol-3(2H)-on.

Analog Beispiel 21 aus:

5,79 g 2-Chlorseleno-3-methylbenzoesäurechlorid

1,46 g tert-Butylamin

Ausbeute: 2,58 g (44,6% d.Th.) Fp. 202—205°C

Beispiel 30

2-tert-Butyl-7-nitro-1,2-benzisoselenazol-3(2H)-on.

Analog Beispiel 21 aus:

6,0 g 2-Chlorseleno-3-nitrobenzoesäurechlorid

1,46 g tert-Butylamin

Ausbeute: 2,8 g (50,2% d.Th.) Fp. 150°C

**Patentansprüche für die Vertragsstaaten: BE CH DE FR GB IT LI LU NL SE**

1. Benzisoselenazolonyl-Derivate der allgemeinen Formel I

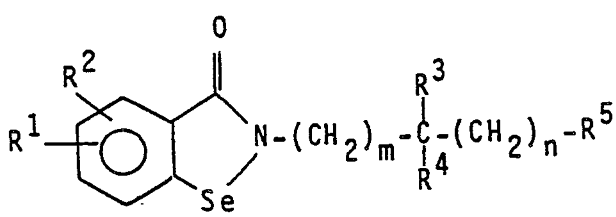

I

worin

$R^1$, $R^2$ gleich oder verschieden sind und unabhängig voneinander Wasserstoff, Halogen, $C_{1-4}$-Alkyl, $C_{1-4}$-Alkoxy, Trifluormethyl, Nitro oder zusammen Methylendioxy bedeuten und

$R^3$ für Wasserstoff, $C_{1-4}$-Alkyl gerade oder verzweigt, Mercapto-$C_{1-2}$-alkyl, Methylthio-$C_{1-2}$-alkyl oder Phenylmethyl steht und

$R^4$ Wasserstoff oder Methyl bedeutet, während

$R^5$ für Wasserstoff, Methyl, $C_{3-8}$-Cycloalkyl, —COOH, —$CONH_2$, —CN oder —$COOR^6$ steht, wobei $R^6$ einen geraden oder verzweigten $C_{1-4}$-Alkylrest bedeutet und

m, n gleich oder verschieden sind und Null oder eine ganze Zahl von 1—8 sind, wobei die Alkylenkette

$-(CH_2)_n-$ bei n = 6 auch als 1,4-Cyclohexylengruppe vorliegen kann, ausgenommen Verbindungen der Formel I, in denen der Rest

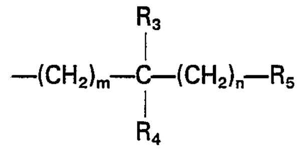

eine n-Alkylgruppe mit 1—4 C-Atomen ist und dabei gleichzeitig $R^1$ und $R^2$ Wasserstoff ist.

2. Benzisoselenazolonyl-Derivate gemäß Formel I, Anspruch 1, worin

$R^1$, $R^2$ gleich oder verschieden sind und unabhängig voneinander Wasserstoff, Fluor, Chlor, Brom, Methyl, Methoxy, Trifluormethyl, Nitro oder zusammen Methylendioxy bedeuten und

$R^3$ für Wasserstoff, $C_{1-4}$-Alkyl gerade oder verzweigt, Mercapto-$C_{1-2}$-alkyl, Methylthio-$C_{1-2}$-alkyl oder Phenylmethyl steht und

$R^4$ Wasserstoff oder Methyl bedeutet, während

$R^5$ für Wasserstoff, Methyl, $C_{3-8}$-Cycloalkyl-, —COOH, $-CONH_2$ oder $-COOR^6$ steht, wobei $R^6$ einen Methyl-, Ethyl oder tert-Butylrest bedeutet und

m, n gleich oder verschieden sind und Null oder eine ganze Zahl von 1—8 sind.

3. Verfahren zur Herstellung von Verbindungen gemäß den Ansprüchen 1—2, dadurch gekennzeichnet, daß man in an sich bekannter Weise o-Chlorselenobenzoesäurechloride der allgemeinen Formel II

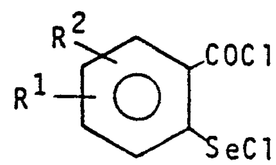 II

worin $R^1$, $R^2$ die in Formel I angegebenen Bedeutungen haben, mit Verbindungen der allgemeinen Formel III

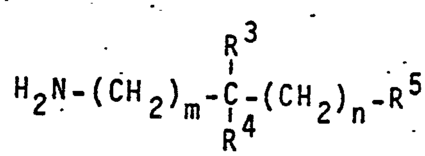 III

worin $R^3$, $R^4$, $R^5$ die in Formel I angegebenen Bedeutungen haben und vorhandene reaktionsfähige Gruppen vorher in üblicher Weise geschützt wurden, unter Ringschlußbedingungen zu den Benzisoselenazolonyl-Derivaten der allgemeinen Formel I umsetzt.

4. Verfahren gemäß Anspruch 3 zur Herstellung von Verbindungen gemäß den Ansprüchen 1—2 mit $R^5$ = COOH, dadurch gekennzeichnet, daß man in einem Eintopfverfahren die Aminocarbonsäuren der Formel III des Anspruches 3 in einem geeigneten Lösungsmittel unter Rückflußbedingungen in die Trimethylsilylester umwandelt, diese nach Abkühlung auf 0°C unter Zugabe des entsprechenden o-Chlorselenobenzoesäurechlorids und der notwendigen Menge Trimethylamin zu den Benzisoselenazolonylalkansäuretrimethylsilylestern kondensiert und aus den erhaltenen Produkten die Trimethylsilylestergruppe in üblicher Weise unter Erhalt der freien Carbonsäuren der Formel I mit $R^5$ = COOH abspaltet.

5. Pharmazeutische Präparate, dadurch gekennzeichnet, daß sie eine Verbindung der Formel I gemäß den Ansprüchen 1—2 als Wirkstoff im Gemisch mit üblichen pharmazeutischen Hilfs- und Trägerstoffen enthalten.

**Patentansprüche für Vertragsstaat: AT**

1. Verfahren zur Herstellung von Benzisoselenazolonyl-Derivaten der allgemeinen Formel I

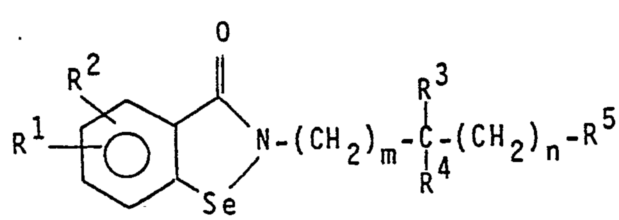 I

worin

$R^1$, $R^2$ gleich oder verschieden sind und unabhängig voneinander Wasserstoff, Halogen, $C_{1-4}$-Alkyl, $C_{1-4}$-Alkoxy, Trifluormethyl, Nitro oder zusammen Methylendioxy bedeuten und

$R^3$ für Wasserstoff, $C_{1-4}$-Alkyl gerade oder verzweigt, Mercapto-$C_{1-2}$-alkyl, Methylthio-$C_{1-2}$-alkyl oder Phenylmethyl steht und

$R^4$ Wasserstoff oder Methyl bedeutet, während

$R^5$ für Wasserstoff, Methyl, $C_{3-8}$-Cycloalkyl, —COOH, —$CONH_2$, —CN oder —$COOR^6$ steht, wobei $R^6$ einen geraden oder verzweigten $C_{1-4}$-Alkylrest bedeutet und

m, n gleich oder verschieden sind und Null oder eine ganze Zahl von 1—8 sind, wobei die Alkylenkette —$(CH_2)_n$— bei n = 6 auch als 1,4-Cyclohexylengruppe vorliegen kann,

ausgenommen Verbindungen der Formel I, in denen der Rest

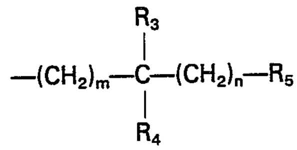

eine n-Alkylgruppe mit 1—4 C-Atomen ist und dabei gleichzeitig $R^1$ und $R^2$ Wasserstoff ist,

dadurch gekennzeichnet, daß man in an sich bekannter Weise o-Chlorselenobenzoesäurechloride der allgemeinen Formel II

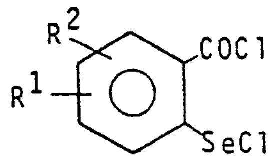

II

worin $R^1$, $R^2$ die in Formel I angegebenen Bedeutungen haben, mit Verbindungen der allgemeinen Formel III

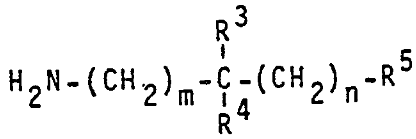

III

worin $R^3$, $R^4$, $R^5$ die in Formel I angegebenen Bedeutungen haben und vorhandene reaktionsfähige Gruppen vorher in üblicher Weise geschützt wurden, unter Ringschlußdingungen zu den Benzisoselenazolonyl-Derivaten der allgemeinen Formel I umsetzt.

2. Verfahren gemäß Anspruch 1 zur Herstellung von Verbindungen der Formel I mit $R^5$ = COOH, dadurch gekennzeichnet, daß man in einem Eintopfverfahren die Aminocarbonsäure der Formel III in einem geeigneten Lösungsmittel unter Rückflußbedingungen in die Trimethylsilylester umwandelt, diese nach Abkühlung auf 0°C unter Zugabe des entsprechenden o-Chlorselenobenzoesäurechlorids und der notwendigen Menge Trimethylamin zu den Benzisoselenazolonylalkansäuretrimethylsilylestern kondensiert und aus den erhaltenen Produkten die Trimethylsilylestergruppe in üblicher Weise unter Erhalt der freien Carbonsäuren der Formel I mit $R^5$ = COOH abspaltet.

**Revendications pour les Etats contractants: BE CH DE FR GB IT LI LU NL SE**

1. Dérivés benzoisosélénazolonyliques de formule générale I

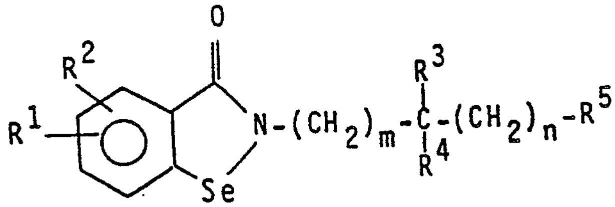

I

dans laquelle

$R^1$, $R^2$, ayant des significations identiques ou différentes, représentent chacun, indépendamment l'un de l'autre, l'hydrogène, un halogène, un groupe alkyle en $C_1$—$C_4$, alcoxy en $C_1$—$C_4$, trifluorométhyle, nitro, ou forment ensemble un groupe méthylène-dioxy, et

$R^3$ représente l'hydrogène, un groupe alkyle droit ou ramifié en $C_1$—$C_4$, un groupe mercapto-alkyle en $C_1$—$C_2$, méthylthio-alkyle en $C_1$—$C_2$ ou phénylméthyle, et

$R^4$ représente l'hydrogène ou un groupe méthyle,

$R^5$ représente l'hydrogène, un groupe méthyle, cycloalkyle en $C_3$—$C_8$, —COOH, —$CONH_2$, —CN ou $COOR^6$ dans lequel $R^6$ représente un groupe alkyle droit ou ramifié en $C_1$—$C_4$, et

m, n, ayant des significations identiques ou différentes, sont chacun égaux à 0 ou à un nombre entier de 1 à 8, la chaîne alkylène —$(CH_2)_n$— pouvant également consister en un groupe 1,4-cyclohexylène lorsque n = 6, à l'exception des composés de formule I dans lesquels le groupe

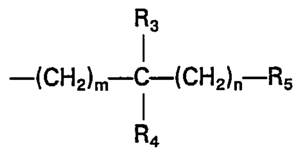

est un groupe n-alkyle en $C_1$—$C_4$, et, simultanément, $R^1$ et $R^2$ représentent des atomes d'hydrogène.

2. Dérivés benzoisosélénazolonyliques de formule I, revendication 1, dans lesquels

$R^1$, $R^2$, ayant des significations identiques ou différentes, représentent chacun, indépendamment l'un de l'autre, l'hydrogène, le fluor, le chlore, le brome, un groupe méthyle, méthoxy, trifluorométhyle, nitro, ou forment ensemble un groupe méthylène-dioxy, et

$R^3$ représente l'hydrogène, un groupe alkyle droit ou ramifié en $C_1$—$C_4$, un groupe mercapto-alkyle en $C_1$—$C_2$, méthylthio-alkyle en $C_1$—$C_2$ ou phénylméthyle, et

$R^4$ représente l'hydrogène ou un groupe méthyle,

$R^5$ représente l'hydrogène, un groupe méthyle, cycloalkyle en $C_3$—$C_8$, —COOH, —$CONH_2$, ou $COOR^6$ dans lequel $R^6$ représente un groupe méthyle, éthyle ou tert-butyle, et

m, n, ayant des significations identiques ou différentes, sont chacun égaux à 0 ou à un nombre entier de 1 à 8.

3. Procédé de préparation des composés selon les revendications 1—2, caractérisé en ce que l'on fait réagir de manière connue en soi des chlorures d'acide o-chlorosélénobenzoïque de formule générale II

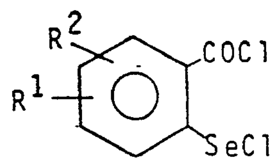

II

dans laquelle $R^1$, $R^2$ ont les significations indiquées en référence à la formule I, avec des composés de formule générale III

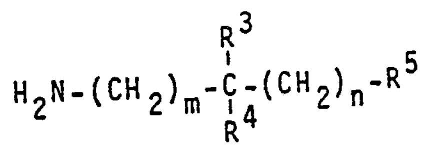

III

dans laquelle $R^3$, $R^4$, $R^5$ ont les significations indiquées en référence à la formule I et les groupes réactifs présents ont été protégés au préalable de la manière habituelle, dans des conditions propres à la cyclisation, ce qui donne des dérivés benzoisosélénazolonyliques de formule générale I.

4. Procédé selon la revendication 3, pour la préparation de composés selon les revendications 1—2 dans lesquels $R^5$ = COOH, caractérisé en ce que, dans une opération en un seul récipient, on convertit les acides aminocarboxyliques de formule III de la revendication 3, dans un solvant approprié, au reflux, en les esters triméthylsilyliques, on refroidit ces derniers à 0°C et on les condense, par addition du chlorure d'acide o-chlorosélénobenzoïque correspondant et de la quantité nécessaire de triméthylamine, en les esters triméthylsilyliques d'acides benzoisoélénazolonylalcanoïques, après quoi on élimine des produits obtenus le groupe ester triméthylsilylique de la manière habituelle, ce qui donne les acides carboxyliques libres de formule I dans laquelle $R^5$ = COOH.

5. Compositions pharmaceutiques, caractérisées en ce qu'elles contiennent en tant que substance active un composé de formule I selon les revendications 1—2, en mélange avec des produits auxiliaires et véhicules pharmaceutiques usuels.

**Revendications pour l'Etat contractant: AT**

1. Procédé de préparation des dérivés benzoisosélénazolonyliques de formule générale I

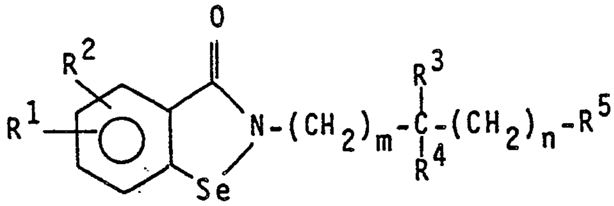

I

dans laquelle

$R^1$, $R^2$, ayant des significations identiques ou différentes, représentent chacun, indépendamment l'un de l'autre, l'hydrogène, un halogène, un groupe alkyle en $C_1$—$C_4$, alcoxy en $C_1$—$C_4$, trifluorométhyle, nitro, ou forment ensemble un groupe méthylène-dioxy,

$R^3$ représente l'hydrogène, un groupe alkyle droit ou ramifié en $C_1$—$C_4$, mercapto-alkyle en $C_1$—$C_2$, méthylthio-alkyle en $C_1$—$C_2$ ou phénylméthyle, et

$R^4$ représente l'hydrogène ou un groupe méthyle,

$R^5$ représente l'hydrogène, un groupe méthyle, cycloalkyle en $C_3$—$C_8$, —COOH, —$CONH_2$, —CN ou $COOR^6$ dans lequel $R^6$ représente un groupe alkyle droit ou ramifié en $C_1$—$C_4$, et

m, n, ayant des significations identiques ou différentes, sont égaux chacun à 0 ou à un nombre entier allant de 1 à 8, la chaîne alkylène —$(CH_2)_n$— pouvant également consister en un groupe 1,4-cyclohexylène lorsque n = 6, à l'exception des composés de formule I dans lesquels le groupe

$$-(CH_2)_m-\overset{R_3}{\underset{R_4}{\overset{|}{\underset{|}{C}}}}-(CH_2)_n-R_5$$

est un groupe n-alkyle en $C_1$—$C_4$, et, simultanément, $R^1$ et $R^2$ représentent d'hydrogène, caractérisé en ce que l'on fait réagir de manière connue en soi des chlorures d'acide o-chlorosélénobenzoïque de formule générale II

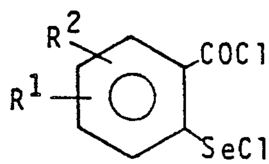

II

dans laquelle $R^1$, $R^2$ ont les significations indiqués en référence à la formule I, avec des composés de formule générale III

$$H_2N-(CH_2)_m-\overset{R^3}{\underset{R^4}{\overset{|}{\underset{|}{C}}}}-(CH_2)_n-R^5 \qquad \text{III}$$

dans laquelle $R^3$, $R^4$, $R^5$ ont les significations indiquées en référence à la formule I et dans lesquels les groupes réactifs présents ont été protégés au préalable de la manière habituelle, dans des conditions propres à une cyclisation, ce qui donne les dérivés benzoisosélénazolonyliques de formule générale I.

2. Procédé selon la revendication 1, pour la préparation de composés de formule I dans laquelle $R^5$ = COOH, caractérisé en ce que, dans une opération en un seul récipient, on convertit les acides aminocarboxyliques de formule III, dans un solvant approprié, au reflux, en les esters triméthylsilyliques, on refroidit ces derniers à 0°C, on condense par addition du chlorure d'acide o-chlorosélénobenzoïque correspondant et de la quantité nécessaire de triméthylamine en les esters triméthylsilyliques d'acides benzoisoélénazolonylalcanoïques et dans les produits obtenus, on élimine de la manière habituelle le groupe ester triméthylsilylique, ce qui donne les acides carboxyliques libres de formule I dans laquelle $R^5$ = COOH.

**Claims for the Contracting States: BE CH DE FR GB IT LI LU NL SE**

1. Benzisoselenazolonyl derivatives of the general formula I

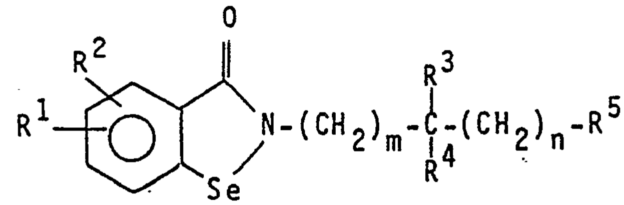

I

wherein

$R^1$, $R^2$ are identical or different and independently represent hydrogen, halogen, $C_{1-4}$-alkyl, $C_{1-4}$-alkoxy, trifluoromethyl, nitro or together mean methylenedioxy,

$R^3$ is hydrogen, straight or branched $C_{1-4}$-alkyl, mercapto-$C_{1-2}$-alkyl, methylthio-$C_{1-2}$-alkyl or phenylmethyl,

$R^4$ is hydrogen or methyl,

$R^5$ is hydrogen, methyl, $C_{3-8}$-cycloalkyl, —COOH, —$CONH_2$, —CN or —$COOR^6$, $R^6$ being a straight or branched $C_{1-4}$-alkyl radical and

m, n are identical or different and are zero or an integer of 1 to 8, wherein the alkylene chain —$(CH_2)_n$— with n = 6 can also be present as a 1,4-cyclohexylene group

with the exception of the compounds corresponding to formula I, wherein the group

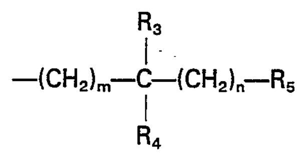

is n-alkyl with 1 to 4 carbon atoms and where both $R^1$ and $R^2$ are hydrogen.

2. Benzisoselenazolonyl derivatives of formula I according to claim 1,

wherein

$R^1$, $R^2$ are identical or different and independently represent hydrogen, fluorine, chlorine, bromine, methyl, methoxy, trifluoromethyl, nitro or together mean methylenedioxy,

$R^3$ is hydrogen, straight or branched $C_{1-4}$-alkyl, mercapto-$C_{1-2}$-alkyl, methylthio-$C_{1-2}$-alkyl or phenylmethyl,

$R^4$ is hydrogen or methyl,

$R^5$ is hydrogen, methyl, $C_{3-8}$-cycloalkyl, —COOH, —$CONH_2$, or —$COOR^6$, $R^6$ being a methyl, ethyl, or tert-butyl radical and

m, n are identical or different and are zero or an integer of 1 to 8.

3. A process for the preparation of compounds according to claims 1 to 2, characterized in that o-chloroseleno benzoic acid chlorides of the general formula II

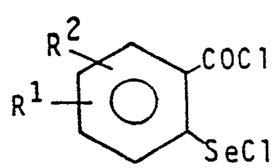

II

wherein $R^1$, $R^2$ have the meanings given in formula I, are reacted with compounds of the general formula III

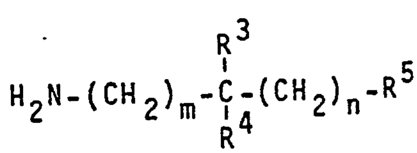

III

wherein $R^3$, $R^4$, $R^5$ have the meanings given in formula I and reactive groups thereof previously being protected in usual manners, under ring-closure conditions in a manner known per se to form the benzisoselenazolonyl derivatives of the general formula I.

4. A process according to claim 3 for the preparation of compounds according to claims 1 to 2 with $R^5$ = COOH, characterized in that the amino carboxylic acids of formula III according to claim 3 in one-pot-process are first converted into the trimethylsilylesters in a suitable solvent under reflux conditions, which silylesters after cooling to 0°C are reacted with the added corresponding o-chloroselenobenzoic acid

chloride and the necessary amount of trimethylamine and thus are condensed to the benzisoselenazolonyl alkanoic acid trimethylsilylesters and finally from the resulting products the protective trimethylsilylester group is split off in usual manners to yield the free carboxylic acids of formula I with $R^5$ = COOH.

5. Pharmaceutical preparations, characterized in that they contain a compound of formula I according to claims 1 to 2 as active component in admixture with common pharmaceutical excipients an carriers.

**Claims for Contracting State: AT**

1. Process for producing benzisoselenazolonyl derivatives of the general formula I

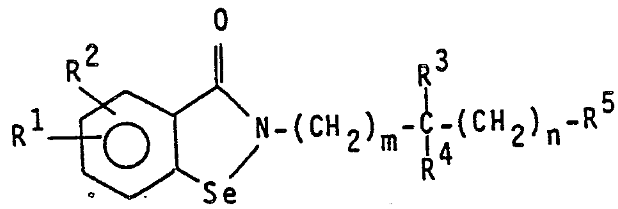

I

wherein

$R^1$, $R^2$ are identical or different and independently represent hydrogen, halogen, $C_{1-4}$-alkyl, $C_{1-4}$-alkoxy, trifluoromethyl, nitro or together mean methylenedioxy,

$R^3$ is hydrogen, straight or branched $C_{1-4}$-alkyl, mercapto-$C_{1-2}$-alkyl, methylthio-$C_{1-2}$-alkyl or phenylmethyl,

$R^4$ is hydrogen or methyl,

$R^5$ is hydrogen, methyl, $C_{3-8}$-cycloalkyl, —COOH, —$CONH_2$, —CN or —$COOR^6$, $R^6$ being a straight or branched $C_{1-4}$-alkyl radical and

m, n are identical or different and are zero or an integer of 1 to 8, wherein the alkylene chain —$(CH_2)_n$— with n = 6 can also be present as a 1,4-cyclohexylene group

with the exception of the compounds corresponding to formula I, wherein the group

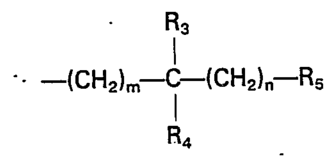

is n-alkyl with 1 to 4 carbon atoms and where both $R^1$ and $R^2$ are hydrogen, characterized in that o-chloroseleno benzoic acid chlorides of the general formula II

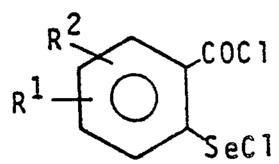

II

wherein $R^1$, $R^2$ have the meanings given in formula I, are reacted with compounds of the general formula III

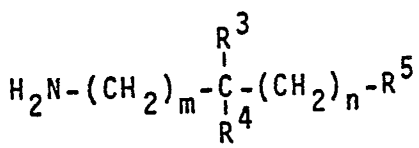

III

wherein $R^3$, $R^4$, $R^5$ have the meanings given in formula I and reactive groups thereof previously being protected in usual manners, under ring-closure conditions in a manner known per se to form the benzisoselenazolonyl derivatives of the general formula I.

2. A process according to claim 1 for the preparation of compounds according to claims 1 to 2 with $R^5$ = COOH, characterized in that the amino carboxylic acids of formula III according to claim 3 in a one-pot-process are first converted into the trimethylsilylesters in a suitable solvent under reflux conditions, which silylesters after cooling to 0°C are reacted with the added corresponding o-chloroselenobenzoic acid chloride and the necessary amount of trimethylamine and thus are condensed to the benzisoselenazolonyl alkanoic acid trimethylsilylesters and finally from the resulting products the protective trimethylsilylester group is split off in usual manners to yield the free carboxylic acids of formula I with $R^5$ = COOH.